# EUROPÄISCHE PATENTSCHRIFT

(11) **EP 0 824 899 B1**
(45) Veröffentlichungstag und Bekanntmachung des Hinweises auf die Patenterteilung: **02.05.2003**
(21) Anmeldenummer: 96202295.0
(22) Anmeldetag: 16.08.1996
(51) Int. Cl.: A61F 2/06

(54) **Endoprothese**
Endoprothesis
Endoprothèse

(43) Veröffentlichungstag der Anmeldung: 25.02.1998
(73) Patentinhaber: Schneider (Europe) GmbH, 8180 Bülach (CH)
(72) Erfinder: Gianotti, Marc, 8542 Wiesendangen (CH)
(74) Vertreter: Kiliaridis, Constantin

(56) Entgegenhaltungen:
- EP-A- 0 712 614
- WO-A-92/06734
- WO-A-94/24961
- US-A- 5 122 154

## Beschreibung

Die Erfindung bezieht sich auf eine Endoprothese zur inneren Stützung von Hohlorganen in Lebewesen, die eine flexible, rohrförmige Gitterstruktur mit stirnseitigen Enden und eine elastische, rohrförmige Hülle aufweist, wobei die Gitterstruktur aus sich in Umfangsrichtung erstreckenden Maschenreihen aufgebaut ist, welche aus aneinandergereihten, von Gittermaterial eingegrenzten Freiräumen bestehen.

Stirnseitige Enden von Endoprothesen der eingangs genannten Art bergen für ein Hohlorgan, in dem eine solche implantiert werden soll, oft ein Verletzungsrisiko in sich. Je nach Bauart weisen diese beispielsweise spitze Drahtenden bei aus Drähten geflochtenen Gitterstrukturen oder auch scharfe Kanten bei aus Blechröhrchen ausgestanzten oder freigeäzten Gitterstrukturen auf. Dadurch kann das Gewebe eines gestützten Hohlorgans vor allem bei Bewegungen der Endoprothese, etwa beim Verschieben der Endoprothese zur Lagekorrektur oder deren Entfernung, oder des Hohlorgans, etwa durch die Peristaltik beispielsweise der Speiseröhre, traumatisch verletzt werden.

Aus der EP 0 621 015 A1 ist eine Endoprothese mit einer zylindrischen Wand aus in Maschen angeordneten Drähten und mit einer Hüllschicht aus elastischem Material, die sich über einen Teil der Länge der Endoprothese erstreckt, bekannt. Da die Hüllschicht unmittelbar mit dem stirnseitigen Ende der Endoprothese abschliesst, bietet diese keinen ausreichenden Schutz vor spitzen oder scharfkantigen Abschlüssen eines stirnseitigen Endes der zylindrischen Wand. Bei radialer Komprimierung der Endoprothese zum Einführen in ein zu stützendes Hohlorgan oder bei axialer Stauchung der implantierten Endoprothese durch peristaltische Bewegungen des Hohlorgans kann die Hüllschicht durchstossen oder durchtrennt werden, so dass verletzende Abschlussteile freigelegt werden. Dann kann ein atraumatisches Verschieben der Endoprothese im Hohlorgan nicht mehr erreicht werden.

Der Erfindung liegt nun die Aufgabe zugrunde, eine Endoprothese der eingangs genannten Art dergestalt weiterzuentwickeln, dass ein durch diese von innen gestützes Hohlorgan vor Verletzungen durch spitze oder scharfkantige Abschlussteile an einem stirnseitigen Ende der Gitterstruktur geschützt ist. Darüberhinaus soll eine Möglichkeit geschaffen werden, die Lage einer gattungsgemässen Endoprothese im Hohlorgan zu korrigieren bzw. diese vollständig aus dem Hohlorgan zu entfernen, ohne das Hohlorgan dabei zu verletzen.

Die Aufgabe wird gemäss der Erfindung gelöst durch eine Endoprothese der eingangs genannten Art mit den Merkmalen des kennzeichnenden Teils des Patentanspruchs 1. Indem die elastische, rohrförmige Hülle im Bereich eines stirnseitigen Endes einer Gitterstruktur aus einem die Gitterstruktur zumindest teilweise umschliessenden Einbettungsteil und aus einem sich daran anschliessenden, über das stirnseitige Ende hinausragenden Überstand besteht, sind verletzende spitze Drahtenden und scharfe Kanten am Stirnende einer Endoprothese durch ein ausreichendes, überstehendes Materialpolster abgesichert, welches gegen Verlust durch den mit der Gitterstruktur verbundenen Einbettungsteil gesichert ist. Insbesondere bei Implantation einer erfindungsgemässen Endoprothese in ein Hohlorgan, das peristaltische Bewegungen in Form von axialen Stössen gegen ein stirnseitiges Ende der Endoprothese ausführt, wie etwa die Speiseröhre, bietet der durch diese Stösse zum Teil umgestülpte Überstand eine zusätzliche Schutzschicht. Daneben vermindert die Hülle aus Einbettungsteil und Überstand das Fremdkörpergefühl für einen Patienten, in dessen Speiseröhre eine solche Endoprothese eingesetzt wurde, welches durch freiliegende, die Innenwand der Speiseröhre berührende Drahtenden oder ähnliches verstärkt wird. Besonders vorteilhaft erweist sich der Überstand der Hülle als verhältnismässig gut zu fassender Ansatzpunkt für Greifwerkzeuge, um die Lage der Endoprothese zum Beispiel in proximale Richtung korrigieren zu können. Damit ist auch eine Möglichkeit für ein vollständiges Entfernen der Endoprothese aus einem Hohlorgan geschaffen.

Weitere Vorteile einer erfindungsgemässen Endoprothese ergeben sich aus bevorzugten Ausführungsformen, die im folgenden anhand der Zeichnungen beschriebenen werden, in deren
- FIG. 1: ein stirnseitiges Ende einer perspektivisch und im axialen Viertelschnitt dargestellten Endoprothese, in
- FIG. 2: ein stirnseitiges Ende einer schematisch in Seitenansicht dargestellten ersten Ausführungsform und in
- FIG. 3: ein vergrösserter Ausschnitt des stirnseitigen Endes einer zweiten Ausführungsform
veranschaulicht ist.

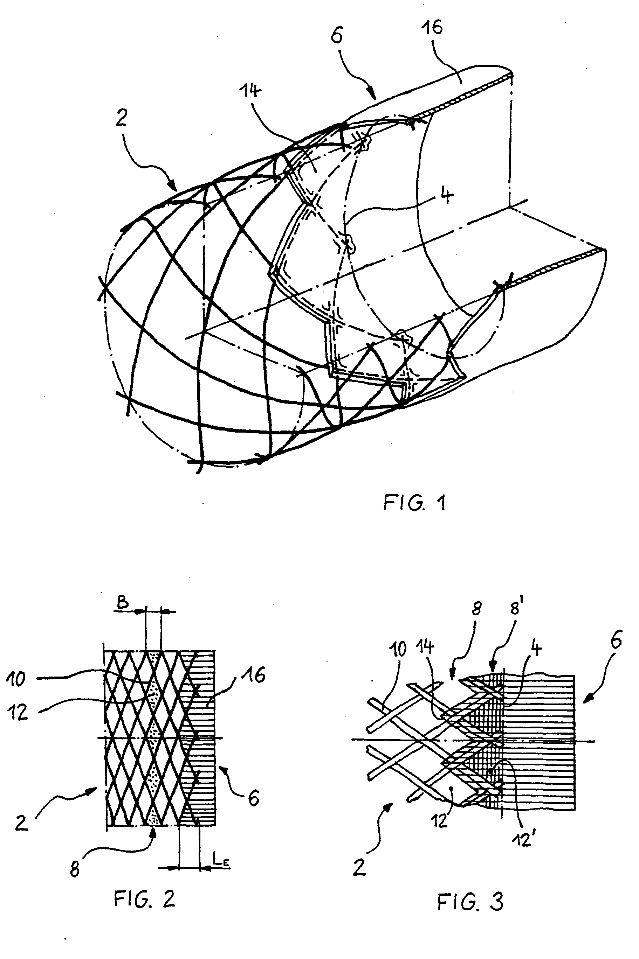

Gemäss FIG. 1 weist die Gitterstruktur 2 einer erfindungsgemässen Endoprothese an einem stirnseitigen Ende 4 eine rohrförmige Hülle 6 auf, welche aus einem die Gitterstruktur 2 teilweise umschliessenden Einbettungsteil 14 und aus einem sich daran anschliessenden, über das stirnseitige Ende hinausragenden Überstand 16 besteht.

FIG. 2 zeigt schematisch einen Abschnitt aus einer Gitterstruktur 2, die aus sich in Umfangsrichtung erstreckenden Maschenreihen 8 aufgebaut ist, welche aus aneinandergereihten, von Gittermaterial 10 eingegrenzten Freiräumen 12 bestehen. Eine Maschenreihe 8 ist zur Verdeutlichung mit punktierten Freiräumen 12 versehen. Bei der durch Längsschraffur kenntlich gemachten Hülle 6 umschliesst des Einbettungsteils 14 die Gitterstruktur 2 vom stirnseitigen Ende 4 her vollständig über eine Länge L_{E} von mindestens der Breite B einer Maschenreihe 8. Dadurch ist gewährleistet, dass die Hülle 6 formschlüssige an der Gitterstruktur 2 gesichert ist, womit die Hülle 6 nicht durch die Bewegungen der Endoprothese in einem Hohlorgan von der Gitterstruktur 2 abgezogen werden und verloren gehen kann. Die Länge des Überstandes 16 ist hier ebenfalls in der Grössenordnung einer Breite B einer Maschenreihe 8. So bietet er einerseits eine hinreichend starke Schutzschicht zwischen spitzen Drahtenden und der Innenwand des Hohlorgans und andererseits genügend Angriffsfläche, um ein Greifwerkzeug anklemmen zu können.

In einer in FIG. 3 dargestellten vorteilhaften Ausführungsform umschliesst der Einbettungsteil 14 vom stirnseitigen Ende 4 her Maschenreihen 8', karriert gekennzeichnet, aus von Gittermaterial 10 mit Unterbrechung eingegrenzten Freiräumen 12' derart vollständig, dass die Freiräume 12 der ersten Maschenreihe 8 aus von Gittermaterial 10 ohne Unterbrechung eingegrenzten Freiräumen 12 nicht mehr vom Einbettungsteil 14 überzogen sind. Hierdurch erzielt man bei sicherer Verankerung der Hülle 6, dass die fixierende Wirkung der Hülle 6 auf die Gitterstruktur 2 verringert und demzufolge Flexibilität und Komprimierbarkeit der Endoprothese im Bereich des stirnseitigen Endes 4 nur wenig eingeschränkt werden.

Gemäss einer bevorzugte Ausgestaltung der Erfindung weist die Hülle 6 hier nicht dargestellte Kanäle auf, die das Gittermaterial 10 dicht umschliessen und entlang derer das Gittermaterial 10 verschiebbar ist. Dies ermöglicht ein Anpassen der Hülle 6 an die Verformungen der Gitterstruktur 2 durch Bewegungen der Endoprothese infolge axialer Stauchung und Verbiegung oder radialer Kompression und Expansion. Durch Verschieben des Einbettungsteils 14 entlang des Gittermaterial 10 kann die elastische Hülle 6 jeweils den Zustand minimaler Spannung einnehmen und vermindert so die Riss- oder Durchstossungsgefahr der Hülle 6.

Bei einer aus Drähten 10 geflochtenen Gitterstruktur 2 weist die Hülle 6 im Mittel eine Wandstärke von etwa der Hälfte eines Durchmessers der Drähte 10 auf. Diese Wandstärke hat sich als hinsichtlich der Reissfestigkeit und des Materialverbrauchs der Hülle 6 als zweckmässig erwiesen. Als Vorteil hat sich die Hülle 6 am proximalen stirnseitigen Ende 4 von geflochtenen Gitterstrukturen 2 als Entflechtungsschutz erwiesen, der beispielsweise bei Einhaken in das Geflecht mit einem Greifwerkzeug distal vom Einbettungsteil 14 und Ziehen der Endoprothese in proximale Richtung wirksam ist.

Die Hülle 6 einer erfindungsgemässen Endoprothese wird bevorzugt aus einem Polyurethanelastomer hergestellt. Wegen seiner Biokompatibilität wird beispielsweise das unter der Marke ChronoFlex™ von der Firma PolyMedica Industries vertriebene Polyurethanelastomer verwendet.

### Bezugszeichenliste

- 2: Gitterstruktur
- 4: stirnseitiges Ende
- 6: Hülle
- 8: Maschenreihe (vollständiger Maschen)
- 8': Maschenreihe (unvollständiger Maschen)
- 10: Gittermaterial
- 12: Freiraum (vollständiger Maschen)
- 12': Freiraum (unvollständiger Maschen)
- 14: Einbettungsteil
- 16: Überstand

- B: Breite einer (vollständigen) Maschenreihe 8
- L_{E}: Länge des Einbettungsteils 14

## Patentansprüche

1. Endoprothese zur inneren Stützung von Hohlorganen in Lebewesen, die eine flexible, rohrförmige Gitterstruktur (2) mit stirnseitigen Enden (4) und eine elastische, rohrförmige Hülle (6) aufweist, wobei die Gitterstruktur (2) aus sich in Umfangsrichtung erstreckenden Maschenreihen (8, 8') aufgebaut ist, welche aus aneinandergereihten, von Gittermaterial (10) eingegrenzten Freiräumen (12, 12') bestehen, **dadurch gekennzeichnet, dass** die Hülle (6) im Bereich eines stirnseitigen Endes (4) aus einem die Gitterstruktur (2) zumindest teilweise umschliessenden Einbettungsteil (14) und aus einem sich daran anschliessenden, über das stirnseitige Ende (4) hinausragenden Überstand (16) besteht.

2. Endoprothese nach Anspruch 1, **dadurch gekennzeichnet, dass** der Einbettungsteil (14) die Gitterstruktur (2) vom stirnseitigen Ende (4) her über eine Länge (L_{E}) von mindestens der Breite (B) einer Maschenreihe (8) vollständig umschliesst.

3. Endoprothese nach Anspruch 1 oder 2, **dadurch gekennzeichnet, dass** der Einbettungsteil (14) vom stirnseitigen Ende (4) her Maschenreihen (8') aus von Gittermaterial (10) mit Unterbrechung eingegrenzten Freiräumen (12') derart vollständig umschliesst, dass die Freiräume (12) der ersten Maschenreihe (8) aus von Gittermaterial (10) ohne Unterbrechung eingegrenzten Freiräumen (12) nicht mehr vom Einbettungsteil (14) überzogen sind.

4. Endoprothese nach Anspruch 1, **dadurch gekennzeichnet, dass** die Länge (L_{Ü}) des Überstandes (16) etwa der Breite (B) einer Maschenreihe (8) entspricht.

## Claims

1. Endoprosthesis for internal support of hollow organs in living beings, which endoprosthesis has a flexible tubular lattice structure (2) with end faces (4) and an elastic tubular sheath (6), the lattice structure (2) being made up of peripherally extending mesh rows (8, 8') which consist of juxtaposed free spaces (12, 12') bounded by lattice material (10), **characterized in that** the sheath (6), in the area of one end face (4), comprises an embedding part (14) at least partially enclosing the lattice structure (2) and an adjacent projecting part (16) protruding beyond the end face (4).

2. Endoprosthesis according to Claim 1, **characterized in that** the embedding part (14) completely encloses the lattice structure (2), from the direction of the end face (4), over a length (L_{E}) of at least the width (B) of a mesh row (8).

3. Endoprosthesis according to Claim 1 or 2, **characterized in that**, from the direction of the end face (4), the embedding part (14) completely encloses mesh rows (8') consisting of free spaces (12') bounded with interruption by lattice material (10) in such a way that the free spaces (12) of the first mesh row (8) of free spaces (12) bounded without interruption by lattice material (10) are no longer covered by the embedding part (14).

4. Endoprosthesis according to Claim 1, **characterized in that** the length (L_{U}) of the projecting part (16) corresponds approximately to the width (B) of a mesh row (8).

## Revendications

1. Endoprothèse pour le support interne d'organes creux pour des êtres vivants, qui présente une structure réticulaire (2) flexible tubulaire avec des extrémités de bout (4) et une enveloppe tubulaire élastique (6), la structure réticulaire (2) étant constituée de rangées de mailles (8, 8') s'étendant dans la direction périphérique, qui se composent d'espaces libres (12, 12') en rangées juxtaposées, limités par un matériau grillagé (10), **caractérisée en ce que** l'enveloppe (6), dans la région d'une extrémité de bout (4) se compose d'une partie d'enrobage (14) entourant au moins partiellement la structure réticulaire (2) et d'une projection (16) s'y raccordant, saillant vers l'extérieur au-delà de l'extrémité de bout (4).

2. Endoprothèse selon la revendication 1, **caractérisée en ce que** la partie d'enrobage (14) entoure complètement la structure réticulaire (2) depuis l'extrémité de bout (4) sur une longueur (L_{E}) représentant au moins la largeur (B) d'une rangée de mailles (8).

3. Endoprothèse selon la revendication 1 ou 2, **caractérisée en ce que** la partie d'enrobage (14) entoure complètement, depuis l'extrémité de bout (4), des rangées de mailles (8') constituées par des espaces libres (12') limités, de manière interrompue, par du matériau grillagé (10), de telle sorte que les espaces libres (12) de la première rangée de mailles (8) constituée d'espaces libres (12) limités sans interruption par du matériau grillagé (10), ne soient plus recouverts par la partie d'enrobage (14).

4. Endoprothèse selon la revendication 1, **caractérisée en ce que** la longueur (L_{Ü}) de la projection (16) correspond approximativement à la largeur (B) d'une rangée de mailles (8).
